# EUROPEAN PATENT APPLICATION

(11) **EP 2 604 996 A1**
(43) Date of publication of application: **19.06.2013**
(21) Application number: 11306662.5
(22) Date of filing: 14.12.2011
(51) Int. Cl.: G01N 1/36, E21B 25/00, E21B 49/02, G01N 33/24

(54) **Method for preparing a sample of rock cuttings extracted from a subsoil and associated analysis assembly**

(71) Applicant: Geoservices Equipements, 95700 Roissy en France (FR)
(72) Inventor: Kimour, Farouk, 75017 PARIS (FR); Bondabou, Karim, 34070 MONTPELLIER (FR)
(74) Representative: Rzaniak, Martin

(57) **Abstract**

The method comprises placing a sample of rock on a mold surface (90), forming a solid plug (134) containing the rock sample embedded in a hardened resin matrix.

The method further comprises pouring a UV polymerizable liquid resin on the sample of rock to embed the sample of rock in liquid resin and passing the rock sample embedded in the UV polymerizable liquid resin in a polymerization device (66) comprising a UV source (92) to harden the liquid resin and form the plug (134).

## Description

The present invention concerns a method for preparing a sample of rock cuttings extracted from a subsoil, in particular from a drilling well, the method comprising the following steps :
- placing a sample of rock on a mold surface ;
- forming a solid plug containing the rock sample embedded in a hardened resin matrix.

When drilling an oil well or a well for another effluent (in particular gas, vapor, water), it is known to periodically recover solid samples contained in the drilling mud emerging from the well, in view of their analysis.

The recovered solid samples are visually analyzed to determine geological information on the nature of the formations which are drilled. Additionally, some analysis are carried out to determine the chemical and physical properties of the cuttings, for example the compositional and dimensional properties of the cuttings.

The above-mentioned analyses are carried out either in the vicinity of the well being drilled, for example in a specifically equipped cabin, or in a laboratory dedicated to the study of the cuttings, away from the drilling site.

The correct analysis of the cuttings contributes in determining the location of potential deposits of fluids contained in the formation, in particular by delimiting appropriate geological underground structures.

It is therefore very valuable to provide information on the cuttings as soon as they are recovered from the well by performing on-site analysis.

Considerable progress has been made in the analysis of the cuttings. In particular, very accurate analytical techniques can now be implemented directly on the drilling site, such as x-ray diffraction (XRD), x-ray fluorescence (XRF), microscopy and even nuclear magnetic resonance (NMR).

In order to carry out such analysis, the samples have to be prepared in order to facilitate the handling of the sample in view of its measurement.

Accordingly, in a known method, pieces of rocks, which have been filtered and extracted out of the drilling fluid mud, are embedded in a liquid resin. The liquid resin is subsequently polymerized to form a solid plug, in which the rock pieces are totally contained in a matrix of transparent resin. The plug is subsequently polished to form a flat surface.

In order to form the plug, the rock pieces are impregnated in liquid resin in a mold. The mold is then filled in with liquid resin to encapsulate the cuttings.

The resin is usually a bi-component resin such as an epoxy resin or a polyester resin comprising a monomer and a catalyst.

The two components of the resin are mixed together in the mold, to initiate polymerization of the resin.

One major drawback of this technique is the polymerization time needed for obtaining a solid plug. Indeed, with epoxy resin, the full polymerization time varies from 3 to 12 hours. With polyester resin, this time can be shortened to about an hour and a half. With some acrylics resins, the polymerization time can be further reduced to about 10 minutes.

Since in drilling operations, the sampling frequency generally varies from two to eight samples by hour, it is impossible to get a real time information on the nature of the cuttings. This lack of information leads to delays in analyzing the course of the drilling of the well.

Typically, information concerning the petrophysical properties (porosity) and mineralogical information (mineralogical quantification, fossil recognition) of the cuttings would be very valuable if obtained in real time, i.e. in the vicinity of the well and within a time less than the sampling period.

Additionally, when mixing the two components of the resin, it is difficult to obtain a uniform viscous liquid that will homogenously fill the mold. It is even sometimes tedious to completely fill the voids between the pieces of rock constituting the rock sample. The mixing of the two components can also generate air bubbles which create interferences in further analysis.

Additionally, most of the resins cited above generate toxic gases when they are handled and during polymerization. Such resins are therefore not easy to handle in the confined space of a logging cabin.

Another difficulty is to create a plug which is hard enough to be subsequently polished. In this regard, some resins, such as silicon resin, are not appropriate since the cured resin is too soft for carrying out the polishing operation.

One aim of the invention is therefore to obtain a method for preparing a sample of rock which can be analyzed promptly, the method being very fast and easy to operate, in particular in the vicinity of a drilling well.

One additional aim of the invention is to obtain a preparation method which leads to samples of a high quality, in particular with no bubbles and readily polishable.

To this aim, the invention relates to a method of the above-mentioned type, **characterized in that** the method comprises :
- pouring a UV polymerizable liquid resin on the sample of rock to embed the sample of rock in liquid resin ;
- passing the rock sample embedded in the UV polymerizable liquid resin in a polymerization device comprising a UV source to harden the liquid resin and form the plug.

The method according to the invention may comprise one or more of the following feature(s) taken isolately, or according to any possible technical combination :
- the pouring of the liquid resin comprises pouring a single liquid component on the sample of rock without adding an additional component.
- the pouring of the liquid resin comprises letting the liquid resin spread on the rock sample and in the voids between the pieces of cuttings of the rock sample without mixing.
- the hardening step comprises:
   - emitting of a UV light having a wavelength between 300 nm and 450 nm ;
   - exposing the liquid resin containing the sample of rock to the emitted UV light.
- the polymerizing device comprises an enclosure receiving the UV source, the pouring of the liquid resin being carried out outside of the enclosure, the method comprising placing the mold surface supporting the sample of rock dispersed in liquid resin into the enclosure.
- it comprises the following steps :
   - providing a hollow mold, the hollow mold having a polymerization cavity delimiting the mold surface ;
   - placing the sample of rock on the mold surface at the bottom of the polymerization cavity ;
   - pouring the liquid resin into the polymerization cavity.
- the mold comprises at least a wall delimiting the polymerization cavity made of a material substantially transparent to the UV light emitted by the UV source, the method comprising at least partially transmitting UV light emitted from the UV source through the substantially transparent wall to the polymerization cavity.
- the UV polymerizable forms a transparent hard matrix embedding the sample of rock after the hardening step.
- the UV polymerizable resin comprises a photoinitiator and a monomer able to be polymerized by free radical polymerization, the hardening step comprising:
   - decomposing the photoinitiator into free radicals by submitting the UV polymerizable resin to UV light emitted by the UV source ;
   - polymerizing the monomer with the free radicals resulting from the decomposition of the photoinitiator to form the hardened solid matrix of the solid plug.
- it comprises exposing the sample of rock embedded in the liquid resin to the UV light emitted by the UV source during a time shorter than 6 minutes, advantageously shorter than 4 minutes.
- it comprises a step of polishing the hardened solid matrix containing the rock sample to delimit a flat surface on the plug.
- it comprises the following steps, prior to placing the sample of rock on a mold surface :
- obtaining a sample of a drilling fluid emerging from a well at a sampling point located in the vicinity of the well ;
- separating the cuttings contained in the drilling fluid from the liquid on a filtering means ;
- sampling the rock sample in the filtering means ;
   and the forming of the plug is carried out in the vicinity if the sampling point.
   - it comprises a step of carrying the solid plug to an analyzer, advantageously chosen among a X-ray fluorescence apparatus, a X-ray diffraction apparatus, a digital imager, a scanning electro-microscope, a NMR apparatus, a back scattered electron microscope or an infrared analyzer.

The invention also concerns an analysis assembly assembly for analyzing a rock sample extracted from a subsoil comprising :
- a device for filtering a sample of rock cuttings out of a fluid ;
- a device for preparing the rock sample of cuttings comprising:
   - a mold surface able to support the sample of rock ;
   - a container containing a UV polymerizable liquid resin able to be poured on the mold surface ;
   - a polymerization device able to harden the liquid resin to form a solid plug containing the rock sample embedded in a solid matrix, the polymerization device comprising a UV source.

The assembly according to the invention may also comprise the following features :
- the mold surface is delimited by a mold comprising at least a wall delimiting the mold surface, the wall being substantially transparent to the UV light emitted by the UV source.
- it comprises an analyzer, in particular chosen among a X-ray fluorescence apparatus, a X-ray diffraction apparatus, a digital imager, a scanning electro-microscope, a NMR apparatus, a back scattered electron microscope or an infrared analyzer.

The invention will be better understood upon reading the following description, taken purely as an example, and made in reference to the appended drawings in which :
- Figure 1 is a schematic view, taken in a vertical section, of a drilling installation provided with a first cuttings analysis assembly according to the invention ;
- Figure 2 is a schematic side view of a first preparation device according to the invention ;
- Figure 3 is a synoptic diagram of the main steps of a first method according to the invention ;
- Figure 4 is a bottom view of a solid plug containing a rock sample obtained through the method according to the invention.
- Figure 5 is a side view of a mold used in the preparation device according to the invention.

In everything which follows, the terms "upstream" and "downstream" are understood with respect to the normal direction of circulation of a fluid in a pipe.

A cuttings analysis assembly according to the invention is used for example in a drilling installation 11 for a fluid production well, such as a hydrocarbon production well.

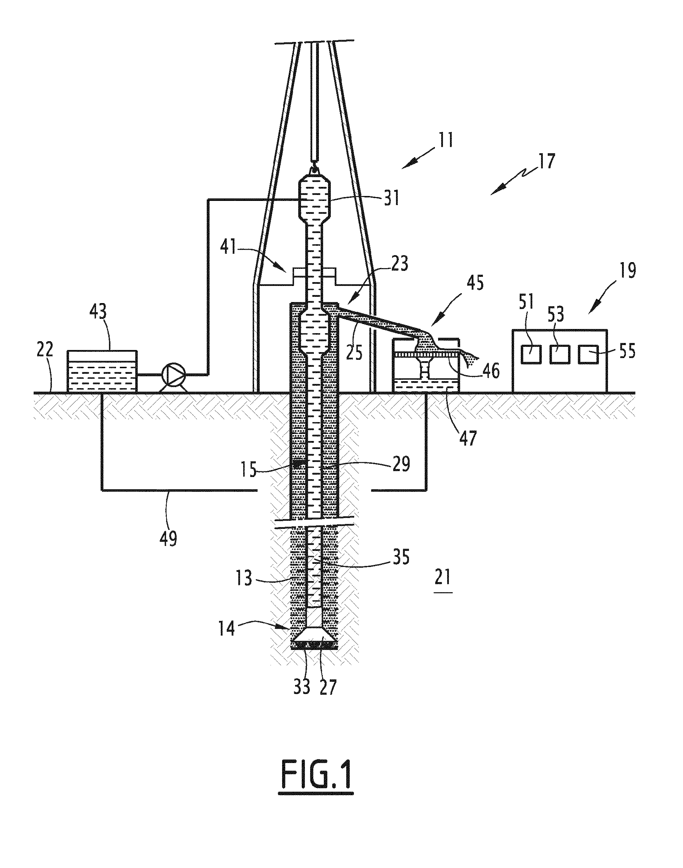

As illustrated in Figure 1, the installation 11 comprises a rotary drilling tool 15 drilling a cavity 14 in the ground, a surface installation 17, where drilling pipes are placed in the cavity 14 and a first cuttings analysis assembly 19 according to the invention.

A well 13 delimiting the cavity 14 is formed in the substratum 21 by the rotary drilling tool 15. At the surface 22, a well head 23 having a discharge pipe 25 closes the well 13.

The drilling tool 15 comprises a drilling head 27, a drill string 29 and a liquid injection head 31.

The drilling head 27 comprises means 33 for drilling through the rocks and/or sediments of the substratum 21, the drilling operation producing solid drilling residues or "cuttings". The drilling head 27 is mounted on the lower portion of the drill string 29 and is positioned at the bottom of the drilling pipe 13.

The drill string 29 comprises a set of hollow drilling pipes. These pipes delimit an internal space 35 which makes it possible to bring a drilling fluid from the surface 22 to the drilling head 27. To this end, the liquid injection head 31 is screwed onto the upper portion of the drill string 29.

The drilling fluid is in particular a drilling mud, in particular a water-based or oilbased drilling mud.

The surface installation 17 comprises means 41 for supporting the drilling tool 15 and driving it in rotation, means 43 for injecting the drilling liquid, and a shale shaker 45, for receiving and treating the effluent emerging from the well.

The injection means 43 are hydraulically connected to the injection head 31 in order to introduce and circulate the drilling fluid in the inner space 35 of the drill string 29.

The shale shaker 45 collects the drilling fluid charged with cuttings which emerges from the discharge pipe 25. The shale shaker 45 is equipped with sieves 46 to allow the separation of the solid drilling residues or cuttings, from the drilling mud.

The shale shaker 45 also comprises a tank 47 located under the sieves 46 to recover the drilling mud deprived of cuttings.

The surface installation 17 further comprises a recirculation duct 49 connecting the recovery tank 47 to the injection means 43 to re-circulate the mud collected in the tank 47 to the injection means 43.

The cuttings analysis assembly 19 is intended for conditioning a rock sample composed of cuttings contained in the drilling mud emerging from the discharge pipe 25, and to analyse the cuttings of the rock sample.

The cuttings are in particular collected at the sieves 46 of the shale shaker 45. These cuttings are made of small pieces of rocks and/or sediments which are generated of the cavity 14.

The average maximal dimension of the cuttings in particular ranges from 0,25 mm to 3 mm, and is generally lower than 2 mm. The cuttings which are analyzed in the analysis assembly 19 generally have a dimension higher than 1 mm.

As will be seen below, the shape of the cuttings can be regular, i.e. of substantially circular or elongated shape, such as ovoid or ellipsoid. Alternatively, the shape of the cuttings can be very irregular.

As shown in Figure 1, the cuttings analysis assembly 19 comprises a sample conditioning unit 51, a sample preparation unit 53 and an analysis unit 55 for determining at least a property of the cuttings contained in the rock sample.

The conditioning unit 51, the sample preparation unit 53 and the sample analysis unit 55 are advantageously located in a specifically equipped cabin in the vicinity of the drilling site.

The sample conditioning unit 51 comprises a cleaning and drying stage, for cleaning and drying the cuttings recovered from the shale shaker 45 and in some cases, a sieving stage for preparing at least two different classes of cuttings by filtering the cuttings according to their maximal dimension on a sieve.

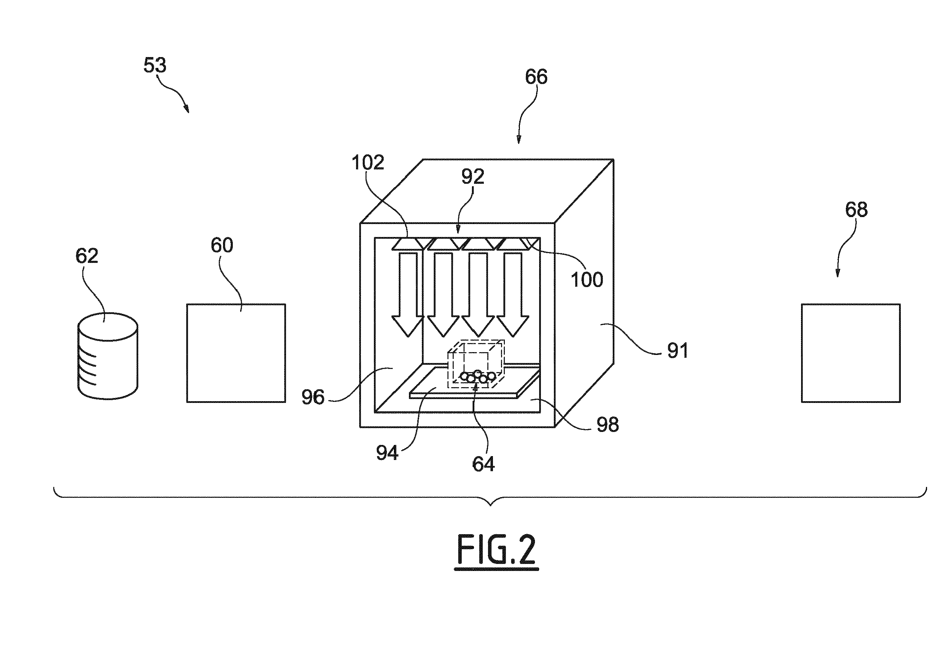

As illustrated in Figure 2, the sample measurement unit 53 comprises a resin container 60, a resin dispensing device 62 and a mold 64 intended to receive the cuttings and a volume of liquid resin.

The sample preparation unit 53 further comprises a polymerizing apparatus 66 and a polishing apparatus 68.

The container 60 receives UV polymerizable resin in liquid form.

Such a liquid resin has a low viscosity, for example lower than 10.000 MPa.s⁻¹ and advantageously from 4.000 MPa.s⁻¹ to 7.000 MPa.s⁻¹ so as to be pourable from the container 60 to the dispensing device 62 and from the dispensing device 62 to the mold 64.

The liquid resin is able to be cured into a hard solid by polymerization under exposure to UV light. It comprises at least a monomer and at least a photoinitiator.

Typically, the liquid resin comprises at least a monomer or a mixture of monomers able to polymerize by free radical polymerization. Example of such monomers comprise acrylate or/and a methacrylate monomers, functional acrylate monomers including but not limited to di-tri and tetra-methacrylates or acrylates.

The mixture may also comprise oligomers such as urethane acrylate oligomers.

The photoinitiator is able to decompose into free radicals to initiate the polymerization of the monomer.

In particular, a typical photoinitiator comprises a carbon-carbon chemical bond which can be broken under UV light to generate two free radicals, each free radical comprising a free electron.

Each free radical reacts with a double or triple carbon-carbon bond in a monomer molecule to create another free radical.

A typical example of photoinitiator comprises an acetophenone group of the type C6H6COCX such as 2,2-diethoxyacetophenone; 2- or 3- or 4-bromoacetophenone; 3- or 4-allyl- acetophenone; 2-acetonaphthone; benzaldehyde; benzoin; the alkyl benzoin ethers; benzophenone; benzoquinone; 1-chloroanthraquinone; p- diacetyl-benzene; 9,10-dibromoanthracene; 9,10-dichloroanthracene; 4,4- dichlorobenzophenone; thioxanthone; isopropyl-thioxanthone; methyl- hioxanthone; [alpha],[alpha],[alpha]-trichloro-para-t-butyl acetophenone; 4-methoxy- enzophenone; 3-chloro-8-nonylxanthone; 3-iodo-7-methoxyxanthone; carbazole; 4-chloro-4'-benzylbenzophenone; fluoroene; fluoroenone; 1 ,4- naphthylphenylketone; 1 ,3-pentanedione; 2,2-di-sec-butoxy acetophenone; dimethoxyphenyl acetophenone; propiophenone; isopropylthioxanthone; chlorothioxanthone; xanthone; and mixtures of any thereof.

Typically, the photopolymerization of the monomer can be activated by exposure to UV light at a wavelength ranging from 100 nm to 600 nm, advantageously from 300 nm to 400 nm, and in a particular example around 365 nm.

The photoinitiator content in the liquid resin is usually less than 10% of the total mass of the liquid resin and is advantageously comprised between 1% and 5% of the total mass, for example 3% of the total mass of the liquid resin.

The liquid resin has generally a flash point above 100°C, a density lower than 1,2 and approximately equal to 1,1. The liquid resin is usually transparent.

When cured, the polymerized resin forms a hard solid matrix which has a hardness at least of 70 Shore D, in particular higher than 75 Shore D and generally comprised between 75 Shore D and 85 Shore D.

The polymerized resin has a water absorption of less than 1% and can undergo temperatures from - 20°C to 120°C, without degradation.

The polymerized resin is transparent to visible light (wavelengths ranging from 400nm to 800nm). As an example, an object embedded on the hard resin matrix can be visually seen through the resin matrix, in particular by a naked eye.

Before use, the liquid resin 62 is usually confined in an opaque container 60 to prevent its polymerization.

The dispensing device 62 comprises for example a mobile vial able to receive the liquid resin poured from the container 60 and to dispense it in the mold 64. Alternatively or additionally, the dispensing device 62 is fixed on the resin container 60 and for example comprises a valve or of a drain tap.

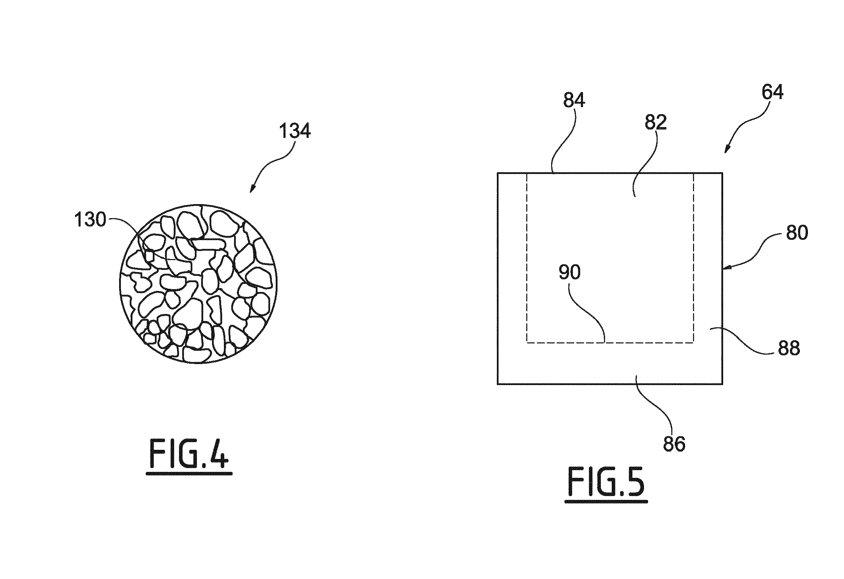

As illustrated in Figures 2 and 5, the mold 64 comprises a cup 80 defining a polymerization cavity 82 opening in an upper opening 84.

In this example, the mold 64 comprises a bottom wall 86 and a lateral wall 88 protruding from the bottom wall 88.

The bottom wall 86 defines an internal mold support surface 90 for laying the pieces of cuttings contained in the rock sample.

In this example, at least a wall 86, 88 of the container is made of a substantially UV transparent material.

By "substantially UV transparent material", it is understood that less than 50%, in particular less than 20% of the quantity of UV light emitted by the polymerization device and passing through the wall 86, 88 absorb in the wall 86, 88.

In the particular example shown in Figures 2 and 5, the mold 64 is made in one piece of a substantially UV transparent material which allows UV light to pass through.

The mold 64 is for example made of a translucent polymer matrix such as polyethylene. Alternatively, the mold is made of another material such as Teflon, silicon, or rubber.

The mold surface 90 is flat and horizontal when placed in the polymerizing apparatus 66.

The polymerization apparatus 66 comprises an enclosure 91, a UV source 92 placed in the enclosure 91 and advantageously a reflector 94 located in the enclosure 91 to face the UV source 92.

The polymerizing apparatus 66 is generally referred to as a "UV polymerization oven".

The enclosure 91 defines an internal space 96 delimited by a lower support surface 98 and by an opposite upper surface 100.

The UV source 92 comprises several UV lamps 102 which, in this example, are fixed on the upper surface 100 to face the lower surface 98.

Each UV lamp 102 is able to emit UV light in a radiation wavelength ranging from 300 nm to 450 nm. The UV lamps 102 are for example made of mercury lamps having emission wavelengths ranging from 100 nm to 600 nm with an emission spectrum comprising at least a peak located between 300 nm and 450 nm.

The lamps have a power input of more than 100 Watts. They are able to deliver a surface power superior to 40 mW/cm² in order to initiate the polymerization of the liquid resin.

The reflector 94 faces the UV source 92 apart from the UV source 92. It is for example made of a metal plate applied on the lower support surface 98. The reflector 94 defines a support surface for the mold 64.

The polishing apparatus 68 comprises a grinding tool for grinding the plug formed in the polymerization device 66, in order to create a flat surface on the plug.

The sample analysis unit 55 comprises at least an analyser. The analyser is for example chosen among a X-ray fluorescence apparatus, a X-ray diffraction apparatus, a digital imager, a microscope, in particular a scanning electron-microscope, a NMR apparatus, a back scattered electron microscope or an infrared analyzer.

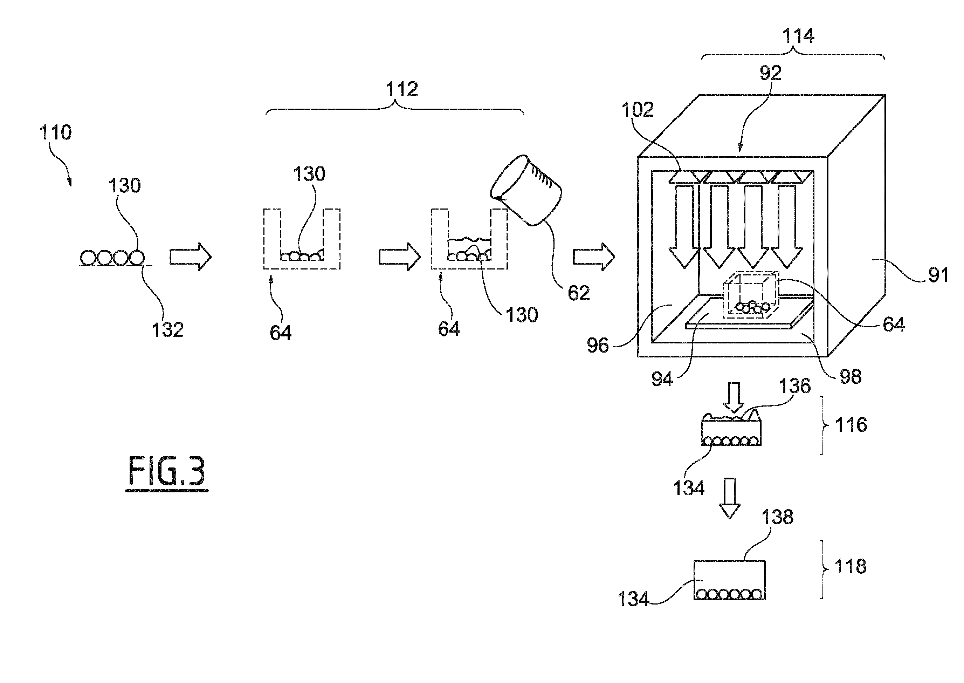

The cuttings preparation and analysis method according to the invention, carried out during the operations of drilling a well, will be now described as an example, with reference to Figures 1 and 3.

In reference to Figure 1, during the drilling operations, the drilling tool 15 is driven in rotation by the surface installation 41. The drilling head 27 drills the rocks and sediments at the bottom of cavity 14 to produce cuttings.

During this operation, a drilling fluid, advantageously a liquid, is continuously introduced into the inner space 35 of the drill string 29 by the injection means 43.

The fluid moves downwards as far as the drilling head 27, and passes into the borehole through the drilling head 27.

The liquid cools and lubricates the drill string 29, and is especially used to evacuate from bottom to surface the cuttings generated during the drilling process. Indeed, the liquid collects the solid cuttings resulting from the drilling operation and moves back upwards through the annular space defined between the drill string 29 and the borehole 13. The liquid charged with solids, in particular cuttings, is subsequently evacuated through the discharge pipe 25.

The liquid charged with solids is then evacuated on the shale shaker 45 to separate the solids from the liquid which carries the solids. The cuttings above a certain side, i.e. higher than 1 mm, are retained on the sieves 46 of the shale shaker 45 and the liquid flows down through the sieves 46 to the tank 47.

At regular intervals, e.g. at a period ranging from 15 minutes to 60 minutes, or at regular depth intervals e.g. ranging from one foot to 15 feet, a rock sample consisting of cuttings 62 is collected on the shale shaker 45.

The sample is taken to the sample conditioning unit 51.

As illustrated in Figure 3, the method according to the invention comprises a first step 110 of conditioning the sample, a second step 112 of dispensing the liquid resin in the mold 64, a third step 114 of polymerization of the liquid resin to form a plug, a fourth step 116 of polishing the plug and a fifth step 118 carrying the polished plug to the analyser.

At step 110, the pieces of cuttings 130 forming the rock sample are cleaned with a cleaning liquid, such as water. The cuttings are then dried. Advantageously, the cuttings are separated according to their sizes on a sieve 132, in particular to remove the smaller cuttings.

At step 112, a single layer of cuttings 130 is laid on the support surface 90 of the mold 64. The thickness of the layer of cuttings is usually lower than 3 mm and comprised between 0,5 mm and 3 mm.

Liquid resin is sampled from the container 60. It is poured in the cavity 82, using the dispensing device 62, to fully embed and cover the cuttings 130 laying on the mold support surface 90.

The thickness of resin added in the mold is generally higher than 0,5 mm and ranges from 0,5 mm to 20 mm.

At least a layer comprising exclusively liquid resin is then formed above the different pieces of cuttings 130.

Moreover, due to its viscosity, the liquid resin totally fills the voids between the different pieces of cuttings 130 without generating bubbles.

Additionally, since the resin is poured as a single liquid component, without additional component, it is not necessary to mix it when it is poured in the dispensing device 62 or when it is introduced in the cavity 82.

On the contrary, the method advantageously comprises a step of letting the resin spontaneously spread on the pieces 130 of cuttings and in the voids between them, without supplying agitation.

The mold 64 containing the cuttings 130 embedded in a liquid resin is then introduced into the enclosure 91, in the internal space 96. The mold 94 is laid on the upper surface of the reflector 94 when available, or on the lower surface 98.

The UV lamps 102 of the UV source 92 are then activated to emit a UV electromagnetic radiation towards the reflector 94.

The UV emitted by the source 92 enters the internal volume 82 through the upper opening 84 and reaches the liquid resin. Additionally, some of the light emitted by the UV source 92 is reflected on the reflector 94 and enters the polymerization cavity 82 through the transparent walls 86, 88.

The UV radiation received by the resin initiates the decomposition of the photoinitiator to generate free radicals. The molecules of photoinitiator pass from a stable state to an excited state. In the excited state, a chemical bond of the photoinitiator splits by homolytic separation or by hydrogen removal to generate the free radicals.

The free radicals generated by the decomposition of the photoinitiator react with the monomer to carry out the polymerization of the liquid resin. The liquid resin cures and becomes solid. In particular, a cross-linked polymer matrix is formed around the cuttings 130, advantageously in the voids delimited between the cuttings 130.

The polymerization is initiated almost immediately.

In order to obtain substantially full polymerization of the resin, the liquid resin is exposed to UV light for a time shorter than ten minutes, possibly shorter than four minutes, in particular between one minute and two minutes.

The final thickness of the polymer can be controlled depending on the chosen penetration of the UV light in the liquid resin.

The mold 94 containing a raw solid plug 134 is then taken out of the enclosure 91. The raw plug 134 is extracted out of the mold 64.

The raw plug 134 may comprise an uneven outer surface 136.

At step 116, at least an uneven outer surface 136 of the plug 134 is polished to form a flat surface 138.

At step 118, the polished plug 134 is carried to the sample analysing unit 55 to be analysed by the analyzer.

Since the polymerization of the resin is carried out extremely rapidly and extremely efficiently, it is possible to prepare a rock sample in less than ten minutes, advantageously in less than six minutes. The rock sample is conditioned in a plug 134 of hard resin able to be directly analyzed by the analyser.

The information delivered by the analyzer can then be provided quickly, which increases the possibilities of adjusting or controlling the ongoing drilling operations.

A real time analysis of the rock samples can then be carried out with simple and easy means.

Additionally, the method according to the invention can be easily implemented in the vicinity of a drilling site, e.g. in an area located less than 100 meters of the point at which the drilling mud is sampled.

Furthermore, the method according to the invention does not require either a mixing or a complex handling of the chemicals involved in the formation of the polymer matrix.

The method according to the invention is therefore safe and reliable.

In a variant, the photo initiator decomposes into ions under exposure to UV light to start the polymerization. The polymerization is an ionic polymerization.

More generally, the method according to the invention also applies to the preparation of rock samples obtained from a mining operation.

## Claims

1. Method for preparing a sample of rock cuttings (130) extracted from a subsoil, in particular from a drilling well, the method comprising the following steps :
- placing a sample of rock on a mold surface (90);
- forming a solid plug (134) containing the rock sample embedded in a hardened resin matrix ;
**characterized in that** the method comprises :
- pouring a UV polymerizable liquid resin on the sample of rock to embed the sample of rock in liquid resin ;
- passing the rock sample embedded in the UV polymerizable liquid resin in a polymerization device (66) comprising a UV source (92) to harden the liquid resin and form the plug (134).

2. Method according to claim 1, **characterized in that** the pouring of the liquid resin comprises pouring a single liquid component on the sample of rock without adding an additional component.

3. Method according to anyone of claims 1 or 2, **characterized in that** the pouring of the liquid resin comprises letting the liquid resin spread on the rock sample and in the voids between the pieces of cuttings of the rock sample without mixing.

4. Method according to any one of the preceding claims, **characterized in that** the hardening step comprises:
- emitting of a UV light having a wavelength between 300 nm and 450 nm ;
- exposing the liquid resin containing the sample of rock to the emitted UV light.

5. Method according to any one of the preceding claims, **characterized in that** the polymerizing device (66) comprises an enclosure (91) receiving the UV source (92), the pouring of the liquid resin being carried out outside of the enclosure (91), the method comprising placing the mold surface (90) supporting the sample of rock dispersed in liquid resin into the enclosure (91).

6. Method according to any one of the preceding claims, **characterized in that** it comprises the following steps :
- providing a hollow mold (64), the hollow mold having a polymerization cavity (82) delimiting the mold surface (90);
- placing the sample of rock on the mold surface (90) at the bottom of the polymerization cavity (82);
- pouring the liquid resin into the polymerization cavity (82).

7. Method according to claim 6, **characterized in that** the mold (64) comprises at least a wall (84, 86) delimiting the polymerization cavity (82) made of a material substantially transparent to the UV light emitted by the UV source (92), the method comprising at least partially transmitting UV light emitted from the UV source (92) through the substantially transparent wall (84, 86) to the polymerization cavity (82).

8. Method according to any one of the preceding claims, **characterized in that** the UV polymerizable forms a transparent hard matrix embedding the sample of rock after the hardening step.

9. Method according to any one of the preceding claims, **characterized in that** the UV polymerizable resin comprises a photoinitiator and a monomer able to be polymerized by free radical polymerization, the hardening step comprising:
- decomposing the photoinitiator into free radicals by submitting the UV polymerizable resin to UV light emitted by the UV source ;
- polymerizing the monomer with the free radicals resulting from the decomposition of the photoinitiator to form the hardened solid matrix of the solid plug (134).

10. Method according to any one of the preceding claims, **characterized in that** it comprises exposing the sample of rock embedded in the liquid resin to the UV light emitted by the UV source during a time shorter than 6 minutes, advantageously shorter than 4 minutes.

11. Method according to any one of the preceding claims, **characterized in that** it comprises a step of polishing the hardened solid matrix containing the rock sample to delimit a flat surface (138) on the plug (134).

12. Method according to any one of the preceding claims, **characterized in that** it comprises the following steps, prior to placing the sample of rock on a mold surface (90) :
- obtaining a sample of a drilling fluid emerging from a well at a sampling point located in the vicinity of the well ;
- separating the cuttings (130) contained in the drilling fluid from the liquid on a filtering means ;
- sampling the rock sample in the filtering means ;
and **in that** the forming of the plug (134) is carried out in the vicinity if the sampling point.

13. Method according to any one of the preceding claims, **characterized in that** it comprises a step of carrying the solid plug (134) to an analyzer, advantageously chosen among a X-ray fluorescence apparatus, a X-ray diffraction apparatus, a digital imager, a scanning electro-microscope, a NMR apparatus, a back scattered electron microscope or an infrared analyzer.

14. Analysis assembly for analyzing a rock sample extracted from a subsoil comprising :
- a device for filtering a sample of rock cuttings out of a fluid ;
- a device (53) for preparing the rock sample of cuttings (130) comprising:
• a mold surface (94) able to support the sample of rock ;
• a container (60) containing a UV polymerizable liquid resin able to be poured on the mold surface (94) ;
• a polymerization device (66) able to harden the liquid resin to form a solid plug (134) containing the rock sample embedded in a solid matrix, the polymerization device (66) comprising a UV source (92) .

15. Assembly according to claim 14, **characterized in that** the mold surface (94) is delimited by a mold (64) comprising at least a wall (86, 88) delimiting the mold surface (94), the wall (86, 88) being substantially transparent to the UV light emitted by the UV source (92).

16. Assembly according to any of claims 14 to 15, **characterize in that** it comprises an analyzer, in particular chosen among a X-ray fluorescence apparatus, a X-ray diffraction apparatus, a digital imager, a scanning electro-microscope, a NMR apparatus, a back scattered electron microscope or an infrared analyzer.
